# EUROPEAN PATENT APPLICATION

(11) **EP 2 623 111 A2**
(43) Date of publication of application: **07.08.2013**
(21) Application number: 11815845.0
(22) Date of filing: 27.09.2011
(51) Int. Cl.: A61K 38/08, C07K 7/06

(54) **PEPTIDES FROM THE VENOM OF THE RHOPALURUS JUNCEUS SCORPION AND PHARMACEUTICAL COMPOSITION**

(30) Priority: 27.09.2010 CU 20100186
(71) Applicant: Grupo Empresarial De Producciones Biofarmaceuticas Y Quimicas, Boyeros, La Habana 17200 (CU)
(72) Inventor: FRAGA CASTRO, José, Antonio, La Habana 11300 (CU); MEDINA GALI, Regla, María, La Habana 11300 (CU); DIAZ GARCIA, Alexis, La Habana 10400 (CU); GUEVARA ORELLANA, Irania, La Habana 19290 (CU); RODRIGUEZ TORRES, Caridad, Clara, La Habana 10500 (CU); RODRIGUEZ COIPEL, Judith, Mayabeque 33500 (CU); RIQUENES GARLOBO, Yanelis, La Habana 10400 (CU); GONZALEZ MARRERO, Isbel, La Habana 10500 (CU); PEREZ CAPOTE, María, Regla, 19290 La Habana (CU)
(74) Representative: Isern-Jara, Nuria
(86) International application number: PCT/CU2011/000006
(87) International publication number: WO 2012/041261

(57) **Abstract**

This invention refers to new peptides obtained from *Rhpalurus junceus* scorpion venom, which has a high content of proteins, lipids, carbohydrates, amino acids, inorganic salts and other ions, including peptides as active principles. The invention also includes a formulation used as a drug due to its anticarcinogenic, analgesic and anti-inflammatory properties which improve the quality of life of cancer patients.

## Description

### Field of the Invention

The present invention is linked essentially to the pharmaceutical industry and particularly to the identification of peptides obtained from the *Rhopalurus junceus* scorpion, Buthidae family, *Rhopalurus* genus, *R. junceus* species, common name "red" scorpion, venom which contains a mix of peptides, proteins, amino acids and free amines.

### Previous art.

Scorpions are land arthropods and, as poisonous animals, they are the oldest group as they were the first ones to develop on Earth. There are approximately over 1 500 scorpion species whose taxonomic classification is *Phylum Arthropoda, Arachnida* Class scorpions. In their natural habitat, the venom of scorpions is an opalescent, milky fluid with pH 7.12 and it contains mucus, lipids, carbohydrates, amino acids, inorganic salts, low-molecular weight organic molecules and a wide variety of proteins with molecular weights ranging 3 kDa-90 kDa which are the main component. Recently, research on scorpion venoms has taken momentum due to their high content of peptides that have shown a wide spectrum of pharmacological activity, for which reason they are invaluable tools for biomedical research (Martin-Eauclaire M-F, Segoard M, Ramos C, Cestele S, Bougis PE, and Svenson B, Production of active insect-specific scorpion neurotoxin in yeast. Eur.J. Biochem. 1994; 223; 637-45; Bednarek MA, Bugianesi RM, Leonard RJ, Felix JP. Chemical synthesis and structure-function studies of margatoxin, a potent inhibitor of voltage dependent potassium channel in human T lymphocytes. Biochem Biophys Res Commun. 1994 Jan 28; 198(2):619-25.) In this sense, recent years have seen a greater number of patent and non-patent publications about the activity of these toxins and their derivatives like anti-inflammatory components (Rajendra W, Armugan A and Jeyaseeian K. Toxins in anti-nociception and anti-inflammation. Toxicon 2004 July; 44(1):1-17); analgesics (Guan RJ, Wang CG, Wang M and Wang DC. A depressant insect toxin with a novel analgesic effect from scorpion Buthus martensii Karsch. Biochem. Biophys. File 2001; 1549(1):9-18), in cancer treatment (Liu YF, Ma RL, Wang SL, Duan ZY, Zhang JH, Wu LJ and Wu CF. Expression of an antitumor-analgesic peptide from the venom of Chinese scorpion Buthus martensi Karsch in Escherichia coli. Protein Expr. Purif. 2003; 27(2):253-8; Wang WX, Ji YH. Scorpion venom induces glioma cell apoptosis in vivo and inhibits glioma tumor growth in vitro. J Neurooncol. 2005 May; 73(1):1-7) and neurodegenerative diseases (Rajendra W, Armugam A and Jeyaseelan K. Toxins in anti-nociception and anti-inflammation. Toxicon 2004 July; 44(1):1-17.) Inventions whose composition include scorpion and/or its venom comprise a wide range of formulations intended for cancer therapy. The authors of the 1993 CN 1073480 and CN 1076858 patent papers refer they have obtained a wine allowing for cancer treatment and prevention, in both cases by mixing scorpion and other plant and animal materials. Scorpions are one of the components of formulations in the form of tablets for treating primary liver cancer (CN 1265901, 2000 and CN 1279088, 2001) and of capsules which inhibit tumor cell growth and can cure cancer in patients (CN 1391941, 2003.) Other patents describe various formulations for cancer treatment (CN 1252321; 2000 CN 1316249, 2001; CN 1399979, 2003.)

There are also patents on the exclusive use of toxins obtained from scorpion venom which act on specific tumor cell types. Early works on scorpion venom toxins referred that a peptide was obtained from the *Leiurus quinquestriatus* Hebrew scorpion. That toxin, which is a 4 KDa peptide named chlorotoxin, has a capacity to bind with the chloride channels expressed in gliomas (primary brain tumors from the glia (De Bin JA, Maggio JE, Strichartz GR. Purification and characterization of chlrotoxin, a chloride channel ligand from venom of the scorpion. Am J Physiol 1993; 264:361-369.) Based on this breakthroughs, in 1999 Ullrich et al. described in US patent 5 905 027 a diagnostic method for treating gliomas. Other works taking the specific action of chlorotoxin on certain cell receptors in gliomas as a basis showed that this toxin is a highly specific marker for other kinds of tumors whose cells have a common embryonic origin with the Central Nervous System (CNS) cells (Lyons SA, O'Neal J, Sontheimer H: Chlorotoxin, a scorpion derived peptide, specifically binds to gliomas and tumors of neuroectodermal origin. GLIA 2002; 39:162-73.) Additionally, recent works have shown that chlorotoxin not only inhibits "in vitro" glioma growth but has a capacity to prevent the invasion and spreading of this kind of tumors into brain regions which have not been damaged due to its specific and selective interaction with methalloproteinases, which are enzymes having to do with the high invasive rate of this kind of cancer (Deshane J, Garner CC and Sontheimer H: Chlorotoxin inhibits glioma cell invasion via matrix metalloproteinase-2. J Biol Chem 2003; 278:4135-44.) In 2004, another patent on the antitumor activity of an isolated toxin from the *Buthus martensis karsh* scorpion on animal-implanted tumors (EP20020774251) was published.

In some cases, there is a wide variety of formulations with the ability to treat or cure cancer under the described patents. Yet, these cases do not prove this quality is unique of some of the types of scorpions and toxins involved in their manufacturing. On the other hand, the described toxins obtained from *Leiurus quinquestriatus* scorpions do not show antitumor activity per se, as in the case of chlorotoxin and *Buthus martensis karsh,* which can be used only to treat certain types of tumors as isolated toxins and not as a mix of toxins in whole venom.

In Cuba, there is a *Rhopalurus junceus* scorpion venom diluted solution whose antitumor activity has been proved empirically in studies. During this work, the solution was administered to domestic animals with spontaneous tumors and tumor reduction and obliteration and good survival were observed (CU 22413 A1.) This invention has distinctive features in the sense there is no knowledge about any pharmaceutical formulation for the therapy and life quality improvement which are claimed by this invention.

### Details description of the invention

The composition and pharmacological properties of *Rhopalurus junceus* scorpion venom and/or its derivatives make it a highly valuable natural drug and its anti-inflammatory, analgesic and anti-carcinogenic effects differentiate it substantially from other similar products in the market. It is a totally natural drug combining high-efficacy components to fight tumor cells (peptides) and others that provide it with analgesic and anti-inflammatory activity. Additionally, it has beneficial effects through the induction of tumor cell apoptosis and through its analgesic and anti-inflammatory properties. These features make it very valuable for improving the quality of life of patients suffering from cancer diseases and their related inflammatory conditions. It has been found that the composition of the natural product resulting from this invention includes a series of low molecular weight proteins with *in vitro* anti-carcinogenic activity whose action confirms potential *in vivo* antitumor activity.

One of the purposes of this invention is determining the composition of and describing the peptides which are the active principles with antitumor activity found in venom. The summaries on qualitative composition, chemical screening, chemical-physical properties and pharmacological activity of scorpion venom are shown in Tables I, II, III, IV, V, VI and VII, respectively.

**Table I. Chemical screening of Rhopalurus junceus whole venom used in formulations of this invention.**

| **Assay** | **Results** |
|---|---|
| Dragendorff Assay | Negative |
| Iron Chloride Assay | Negative |
| Shinoda Assay | Negative |
| Lieberman-Burchard Assay | Negative |
| Ninhydrin Assay | Positive |
| Carbohydrates | Positive |
| Lipids | Positive |

**Table II. Determination of metal ions in Rhopalurus junceus whole venom.**

| Sample | Na (mg/L) | Mg (mg/L) | K (mg/L) | Cu (mg/L) | Zn (mg/L) | Cd (mg/L) | Pb (mg/L) |
|---|---|---|---|---|---|---|---|
| Scorpion venom | 114±21 | 4.12±0.28 | 4.66±0.35 | 0.069±0.006 | 11.18±0.7 | <0.004* | <0.01* |

| | | | | | | | |
|---|---|---|---|---|---|---|---|
| *Heavy metals with levels below the lower limit | | | | | | | |

**Table III. Chemical-physical properties of Rhopalurus junceus scorpion venom used in formulations for this invention.**

| Property | Acceptance index |
|---|---|
| pH | 5-7 |
| Protein content | 5-15 mg/mL |
| Appearance | Opalescent |
| Color | Whitisth |

**Table IV. Table of relative molecular weights (RMW) of proteins in scorpion venom ≥obtained through molecular exclusion using low-pressure liquid chromatography and polyacrylamide gel electrophoresis (SDS-PAGE.)**

| Relative molecular weights |
|---|
| ≥ 72 kDa |
| 60 kDa |
| 45 kDa |
| 30 kDa |
| 14 kDa |
| 8 kDa |
| ≤ 4 kDa |

**Table V. Active principles with toxic activity on tumor cells identified from Rhopalurus junceus whole venom.**

| Peptide | Molecular weight | Amount of amino acids |
|---|---|---|
| RjLB-01 | 544.42 Da | 10 |
| RjLB-03 | 1964.0 Da | 17 |
| RjLB-04 | 4748.14 Da | 43 |
| RjLB-05 | 908.0 Da | 8 |
| RjLB-07 | 707.03 Da | 7 |
| RjLB-08 | 712.42 Da | 17 |
| RjLB-09 | 1203.44 Da | 12 |
| RjLB-14 | 5930.45 Da | 50 |

**Table VI. Summary of toxicological studies**

| **General toxicology** | | ***R. junceus* venom (mg/kg)** | | **Effect** |
|---|---|---|---|---|
| Acute toxicology | | | | |
| Oral route | | 2000 | | N.E. |
| IP route | | 5-20 | | DL₅₀=16,41 mg/kg (M y H) |
| Irritability | | | | |
| Dermal | | - | | N.E. |
| Ophthalmic | | - | | N.E. |
| Mouth mucose | | - | | N.E. |

| Repeat dose toxicity | | | | |
|---|---|---|---|---|
| Repeat oral dose toxicity (28 days) | | 100 | | N.E. |
| Subchronic oral toxicity (90 days) | | 0,1-100 | | N.E. |
| Water and food intake | | | N.E. | |
| Weight gain | | | N.E. | |
| Hematological and biochemical parameters | | | | |
| Relative weight of organs | | | N.E. | |
| Pathological anatomy | | | N.E. | |
| | | | N.E. | |

| **Especial toxicity** | | ***R. junceus* venom (mg/kg)** | | **Effect** |
|---|---|---|---|---|
| Mice bone marrow micronuclei | Oral (acute) | 2000 | | N.E. |
| | Oral (repeat doses, 28 days) | 100 | | N.E. |
| | ip | 4,10-13,13 | | N.E. |

| | | | | |
|---|---|---|---|---|
| Legend: N.E.: No effect | | | | |

**Table VII. Summary of pharmacological studies**

| **Pharmacology** | **Effect** |
|---|---|
| Antiproliferative | Cytotoxicity in epithelial tumor cells |
| Antitumoral | Murine solid tumor growth retardation |
| Antimetastasic | Murine lung metastasis reduction |
| Analgesic | Reduced CNS and peripheral pain |
| Anti-inflammatory | Reduced inflammation after topical, oral and intraperitoneal administration |

The following procedures were carried out in order to determine the composition of the mix in this invention.
- Determination of whole proteins through the Lowry method, modified.
- Chemical screening
- Separation of peptides as per molecular weight groups through low-pressure liquid chromatography separation using a 12 HR 10&30 Superose column.
- Polyacrylamide gel protein electrophoresis (SDS-PAGE.)
- Separation of whole venom and of peptides through high-resolution liquid chromatography using a reverse-phase C18 column.
- Determination of 220nm- and 280nm-wavelength UV spectra.
- Cell viability in normal and tumor cells.
- Analysis of each peptide of interest through mass spectrometry.

According to the results from acute and subchronic toxicity assays done in mice, the product from this invention is non-toxic.

The studies which are described in detail below as examples of the invention product do not limit the scope of this application whatsoever.

In order to carry out the experiments given as examples of work, several scorpions were chosen and their venom was extracted through electrical stimulation and diluted in adequately distilled water. It was later clarified by centrifugation at 10 000 rpm for 15 minutes to eliminate components such as mucus and cell debris. Venom protein content determination was performed through the Lowry method, which showed a 5-15 mg/mL concentration.

### Intraperitoneal (IP) administration route

Assay substance and positive control were administered first. After 30 minutes, a 10-15µL croton oil solution was administered to each surface inside and outside the right ear of animals and the left ear was used as control. After three hours, animals were sacrificed following the usual standards for the species (according to FELASA), both ears were severed and round 8-mm diameter samples were taken with a punch and weighed on an analytical scale.

### Topical administration route

First, the 25-30µL croton oil solution was applied on each of the inner and outer surfaces of the animal's right ear and the left ear was used as control. Then the assay substance was given immediately. After three hours, animals were sacrificed following the usual standards for the species (according to FELASA), both ears were severed and round 8-mm diameter samples were taken with a punch and weighed on an analytical scale.

### Oral administration route

The 25-30µL croton oil solution was administered on each of the inner and outer surfaces of the animal's right ear and the left ear was used as control. Then the assay substance was administered by oral route immediately. After three hours, animals were sacrificed following the usual standards for the species (according to FELASA), both ears were severed and round 8-mm diameter samples were taken with a punch and weighed on an analytical scale.

Topical administration of croton oil provides a proper skin inflammation model for evaluating anti-inflammatory agents. The croton oil active principle is Phorbol-12-Myristate 13-Acetate (PMA), which is a potent pro-inflammatory agent whose epicutaneous application results in histological and biochemical changes including higher vascular permeability and vascular rupturing, leukocytary infiltration, protein C activation and a greater release of arachidonic acid and its metabolites. PMA is also known to be a powerful neutrophil activator and there is wide and powerful blood irrigation in the ear.

### IP administration route

IP administration of the 1-5 mg/kg doses per body weight inhibited swelling induced by croton oil in mice ears (Table VIII), and the 3 mg/kg dose was more effective than the IP administration of 15 mg/kg dexamethasone in experimental animals as it had a higher inflammation inhibiting effect (97% vs 60.86%.) The 5 mg/kg dose did not differ statistically (p > 0.05) from the positive control (dexamethasone.)

**Table VIII. Effect of intraperitoneal administration of R. junceus venom in the mice ear edema model**

| Group | | Extinction of edema (mg) | Inflammation inhibition, % |
|---|---|---|---|
| Control | | 7,043 ± 0,27 | - |
| *R. Junceus* venom | 1 mg/kg | 5,050 ± 1,47 b | 27,12 |
| | 3 mg/kg | 1,483 ± 0,93 c | 97,46 |
| | 5 mg/kg | 4,020 ± 1,41 db | 44,09 |
| 15 mg/kg dexamethasone | | 3,117 ± 1,09 d | 60,86 |

| | | | |
|---|---|---|---|
| Values are the mean ±SD. Groups having at least one common letter do not differ statistically. (p > 0.05.) | | | |

### Oral administration route

Administration of 5-, 10- and 20-mg *R. junceus* venom results in significant ear weight loss compared to the negative control group (Table IX), but it doesn't compare statistically to swelling inhibition induced by dexamethasone, which is a good steroidal anti-inflammatory drug that inhibits phospholipase A₂ (the enzyme that is the route for arachidonic acid.)

**Table IX. Effect of oral administration of R. junceus venom in the ear edema model in mice.**

| Group | | Extinction of edema (m/g) | Inflammation inhibition, % |
|---|---|---|---|
| Control | | 11,28 ± 1,29a | - |
| *R. junceus* venom | 5 mg/kg | 7,68 ± 0,50 c | 35,72 |
| | 10 mg/kg | 8,05 ± 0,57 c | 37,39 |
| | 20 mg/kg | 7,76 ± 1,01 c | 38,02 |
| 15 mg/kg dexamethasone | | 4,87 ± 0,95 b | 53,70 |

| | | | |
|---|---|---|---|
| Values are the mean ±SD. Groups having at least one common letter do not differ statistically. (p > 0.05.) | | | |

### Topical administration route

Topical administration of *R. junceus* venom resulted in auricular edema reduction compared to the control group, as shown in Table X.

**Table X. Effect of topical administration of R. junceus venom in the ear edema model in mice.**

| Group | | Extinction of edema (mg) | Inflammation inhibition, % |
|---|---|---|---|
| Control | | 12,00 ± 0,48 a | - |
| *R. junceus* venom | 10 mg/kg | 8,87 ± 0,66 c | 28,23 |
| | 20 mg/kg | 8,25 ± 0,83 c | 21,70 |
| Bencydamine | | 4,87 ± 1,40 b | 67,60 |

| | | | |
|---|---|---|---|
| Values are the mean ±SD. Groups having at least one common letter do not differ statistically. (p > 0.05.) | | | |

Table XI shows the results from the study that was done. Oral administration of *R. junceus* venom brought a slight granulomatose tissue inhibition that took place compared to the negative control group, which suggests activity in the proliferative phase of inflammation. The two studied product doses had a similar effect.

**Table XI. Effect of R. junceus venom on cotton pellet-induced granulomas in rats.**

| Group | Granuloma weight (mg) | Inhibition, % |
|---|---|---|
| Control | 103,33 ± 5,08 a | - |
| 3 mg/kg dexamethasone | 55,40 ± 14,84 b | 44,09 |
| 10 mg/kg *R. Junceus* venom | 80,60 ± 4,74 c | 20,95 |
| 20 mg/kg *R. Junceus* venom | 83,10 ± 6,52 c | 18,49 |

| | | |
|---|---|---|
| Values are the mean ±SD. Groups having at least one common letter do not differ statistically. (p > 0.05.) | | |

Table XII shows granuloma weight and carmine content values for the different experimental groups. The toxin lowered the granuloma weight 1.32 and 2.05 times compared to the control group (p < 0.05) with its 3- and 5-mg/kg doses, respectively.

Angiogenesis is a key process for tumor growth and metastasis, so having products which can inhibit this complex process is an alternative for treating tumor diseases.

Carmine content is an indicator of the formation of new blood vessels in the air-pouch granuloma. Toxin administration reduced new blood vessel formation in the granulomatose tissue by 39.22% and 69.24% with the 3- 5-mg/kg (IP) doses, respectively, with a significant statistical difference between all experimental groups (p < 0.001.)

**Table XII. Effect of R. Junceus venom administered intraperitonially on granuloma weight and carmine content in adjuvant-induced inflammatory angiogenesis in rats (n=10.)**

| **Group** | | **Granuloma weight (g±SD)** | **Carmine content (A±SD)** |
|---|---|---|---|
| **Control** | | 5,26 ± 1,11^{a} | 0,413 ± 0,014^{a} |
| ***R. junceus* venom** | **3 mg/kg** | 3,59 ± 0,33^{b} | 0,251 ± 0,015^{b} |
| | **5 mg/kg** | 2,56 ± 0,74^{b} | 0,127 ± 0,020^{c} |

| | | | |
|---|---|---|---|
| Groups having at least one common letter do not differ statistically (p > 0.05.) | | | |

A: Absorbance at 490 nm

Table XIII shows the results of the analgesic effect of *R. junceus* venom inoculated intraperitonially to the 3% acetic acid-induced contorsions model in mice.

All groups treated with the different venom doses showed much lower (p < 0.05) abdominal constrictions compared to the negative control group. The 2.5- and 7.5-mg/kg venom doses had a similar effect than that in the group given aspirin, a known analgesic drug. On the other hand, the 5.0 mg/kg *R. Junceus* venom dose showed a higher inhibition (87.74%) than that obtained from the administration of 100 mg/kg aspirin (49.28%.)

**Table XIII. Effect of the intraperitonial administration of R. junceus venom in the acetic acid-induced abdominal contorsions model.**

| **Experimental groups** | **Doses (mg/kg)** | **No. contorsions X±DS** | **% inhibition of No. of contorsions** |
|---|---|---|---|
| CN | - | 36,22 ± 5,11 *^{a}* | ------- |
| ASA | 150 | 18,37 ± 4,63 *^{b}* | 49,28 |
| Venom of R. *junceus* | 1 | 22,25 ± 4,53 *^{b}* | 38,57 |
| | 2,5 | 17,87 ± 5,59 *^{b}* | 50,66 |
| | 5 | 4,44 ± 4,33 *^{c}* | 87,74 |
| | 7,5 | 17,86 ± 5,30 *^{b}* | 50,69 |

| **Experimental groups** | **Dose (mg/kg)** | **No. of contorsions X ± DS** | **Inhibition in the No. of contorsions, %** |
|---|---|---|---|
| NC | - | 36,22 ± 5,11^{a} | ---------- |
| ASA | 150 | 18,37 ± 4,63^{b} | 49,28 |
| *R. junceus* venom | 1 | 22,25 ± 4,53^{b} | 38,57 |
| | 2,5 | 17,87 ± 5,59^{b} | 50,66 |
| | 5 | 4,44 ± 4,33 ^{c} | 87,74 |
| | 7,5 | 17,86 ± 5,30^{b} | 50,69 |

| | | | |
|---|---|---|---|
| NC: Negative control; No: Number ASA: Aspirin Groups having at least one common letter do not differ statistically (p > 0.05.) | | | |

### Oral route administration

As evidenced in Table XIV, all groups which were given various venom doses showed a markedly lower number (p < 0.05) of abdominal constrictions compared to the negative control group. A comparison of the venom doses used with the positive control group (aspirin) showed a marked analgesic effect. Doses of 10-, 15- and 20-mg/kg *R. junceus* venom inhibited abdominal contractions in a way similar to that of aspirin. Whereas the 50-mg/kg dose did not bring about statistically significant differences from the positive control, it attained a 54.78% inhibition of abdominal contorsions in the experimental animals.

**Table XIV. Inhibition of acetic acid-induced abdominal contorsions after the oral administration of R. junceus venom.**

| **Experimental groups** | **Dose (mg/kg)** | **Inhibition in the No. of contorsions, %** |
|---|---|---|
| NC | - | ------ |
| ASA | 150 | 47.01 |
| | 1 | 18,84 |
| | 5 | 32,84 |
| | 10 | 36,23 |
| | 15 | 39,85 |
| | 20 | 43,19 |
| | 50 | 54,78 |

| | | |
|---|---|---|
| NC: Negative control ASA: Aspirin | | |

### ● Thermal analgesia model in mice.

The method described in Drug Discover was used.

OF1 male mice weighing an average 20-25g from the National Center for Laboratory Animal Production (CENPALAB) were used.
(pag. 16)

The animals were placed on a (UGO-Basile) hot plate at a 55°C constant temperature both before and after administering the assay substance. The latency of the nociceptive response in the form of hind leg licking or jumping was measured. Only those animals which showed a nociceptive response within 20 seconds were chosen for the assay. Selection ended at 40 seconds.

After administering a physiological saline solution to the negative control, 4% codeine to the positive control, and the various *R. junceus* venom doses, response latency time in each animal were measured after 2-3 hours.

During this work, nociceptive reactivity to a thermal stimulus in mice was measured using the hot plate assay, which is an acute pain sensitivity test to detect opiod analgesia as well as some kinds of significant hyperanalgesic dorsal spine reactions. A substance is considered to have significant analgesic properties when an animal's normal reaction time doubles with its administration.

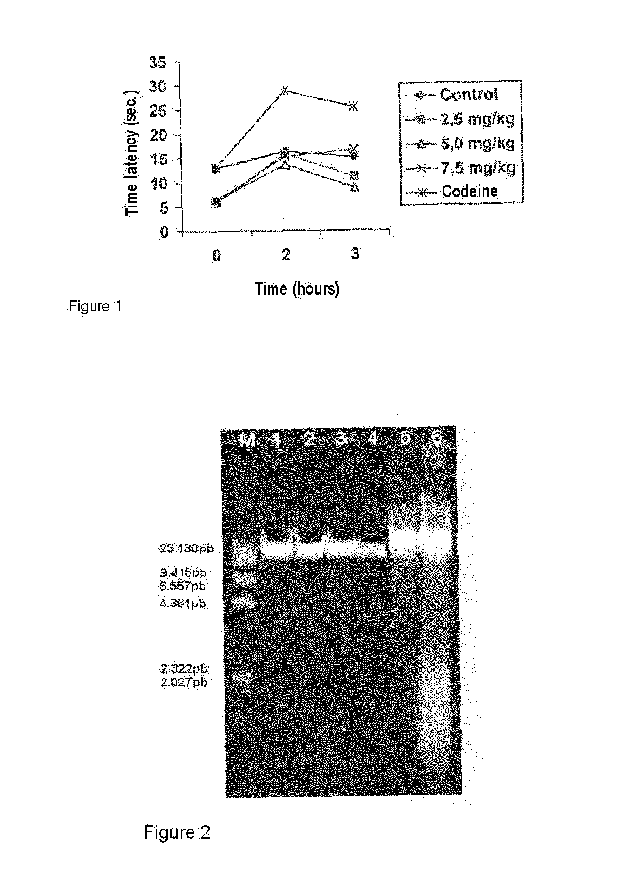

The analgesic effect of *R. junceus* venom is shown in Figure 1 and Table XV. Results indicate that IP administration of three doses of the product significantly reduced hot plate thermal stimulation. Figure 1 shows that the three *R. junceus* venom doses significantly expand nociceptive response latency spans during the hot plate assay with mice.

Pain inhibition in the various experimental groups is shown in Table XV. A powerful heat-induced inhibition of algesia is observed, with the highest inhibition taking place 2 hours after administration. There is a potent effect after three hours, and although 2.5- and 7.5-mg/kg doses have a similar effect on the positive control it is the higher dose which has a longer effect which is greater than that of codeine.

**Table XV. Effect of R. jenceus venom administration in the hot plate model.**

| Group | Dose (mg/kg) | Inhibition, % | |
|---|---|---|---|
| | | 2 h | 3 h |
| Control | - | 26,52 *a* | 16,76 *a* |
| *R. junceus* venom | 2,5 | 173,96 *b* | 92,53 *b* |
| | 5,0 | 109,19 *c* | 36,31 *a* |
| | 7,5 | 145,55 *bc* | 165,54 *c* |
| Codeine | 40 | 120,35 *c* | 94,17 *b* |

| | | | |
|---|---|---|---|
| Groups having at least one common letter do not differ statistically (p > 0.05.) | | | |

The results of the hot plate test evidence that the *R. Junceus* venom has an anti-cociceptive effect on the central nervous system that is higher than the one on the bone marrow as a greater and more complex neuron integration is needed for the licking and jumping response.

**Table XVI. Pro-oxidative potential of R. junceus scorpion venom in the model for Fenanthrolin/Cu2⁺-induced DNA damage.**

| **Assay substance** | **Concentration** (**/mL) | **Extent of damage to DNA** (*****at 532 nm; Median ±SD) |
|---|---|---|
| Negative control | - | 0,069 ± 0,004 |
| *R. junceus* scorpion venom | 7,50 | 0,023 ± 0,009* |
| | 18,70 | 0,36 ± 0,002* |
| | 37,50 | 0,047 ± 0,003* |
| | 56,20 | 0,069 ± 0,012 |
| | 75,0 | 0,075 ± 0,011 |
| Ascorbic acid | 200 µM | 0,130 ± 0.006* |

| | | |
|---|---|---|
| Different letters mean a statistical difference (p < 0.05) compared to control. | | |

**Table XVII. Pro-oxidative potential of R. junceus scorpion venom in the model for Fenanthrolin/Cu2⁺-induced DNA damage.**

| **Assay substance** | **Concentration (µg/mL)** | **Extent of damage to DNA** (OD at 532 nm: Median ± SD) |
|---|---|---|
| Negative control | - | 0.40 ± 0,005 |
| *R. junceus* scorpion venom | 4,50 | 0,024 ± 0,006 |
| | 9,01 | 0,026 ± 0,010 |
| | 18,02 | 0,037 ± 0.008 |
| | 36,04 | 0,024 ± 0,001 |
| | 90,11 | 0,039 ± 0,001 |
| Ascorbic acid | 200 µM | 0,647 ± 0,016* |

| | | |
|---|---|---|
| Different letters mean a statistical difference (p < 0.05) compared to control. | | |

**Table XVIII. Cells used in studies to evaluate the in vitro biological activity of R. junceus scorpion venom.**

| **Cell lines** | **Growth form** | **CC₅₀(mg/mL)** |
|---|---|---|
| HeLa (human cervix carcinoma) | single-layer | 1 |
| HEp-2 (human larynx epidermoid carcinoma) | single-layer | 0,78 |
| NCI-H292 (human lung mucoepidermoid carcinoma) | single-layer | 0,69 |
| A549 (human lung carcinoma) | single-layer | 0,62 |
| U937 (human histiocytic lymphoma) | suspension | ND |
| L929 (murine fibrosarcoma) | single-layer | 1,3 |
| S-180 (murine sarcoma) | suspension | ND |
| F311 (murine mammary adenocarcinoma) | single-layer | 1,2 |
| MRC-5 (human lung fibroblasts) | Single-layer | 2,2 |
| Vero (African green monkey normal kidney cells) | Single-layer | ND |
| MDCK (normal dog kidney cells) | single-layer | ND |
| N₂A (murine neuroblastoma cells) | single-layer | 1,4 |
| Macrophages (extracted from Balc/c mice peritonea) | - | ND |
| Lymphocytes (extracted from Balb/c mice spleens) | suspension | ND |

| | | |
|---|---|---|
| ND non-determined CC₅₀: median cytotoxic concentration | | |

Single-layer (carcinoma) growing cell cultures showed significant cell growth inhibition (p < 0.05) and higher sensitivity than normal cells and tumor cells growing in a suspension.

A comparison between the CC₅₀ of a normal MRC-5 human cell line with human tumor cells showed that *R. junceus* scorpion venom has a differential and significant cytotoxicity (p < 0,05) in tumor cells compared to normal cells (Table XVIII.)

Venom exposure provoked significant growth inhibition (p < 0.05) in human and murine tumor cell lines in single-layer growing conditions. The U937 and S-180 tumor cell lines (both of which grew in suspension) showed less sensitivity to the administration of the venom; the only effect was a slightly lesser growth (Table XVIII.)

Normal cells, lymphocytes and peritoneal macrophages showed less sensitivity than tumor cells. No significant toxic effects were observed (p > 0.05) along the full spectrum of used concentrations (Table XVIII.)

An evaluation of pulmonary metastasis incidence after 50 days showed that the administration of scorpion venom significantly reduced (0.8 mg/kg; 3.2 mg/kg) the onset of spontaneous lung metastases (Table XIX.)

**Table XIX. Incidence of lung metastasis onset in Balb/c mice implanted with F311 murine mammary tumor. *Statistically significant differences from control (p < 0.05.)**

| Group | Number of mice | Metastasis incidence | No. of metastasis nodules (range) |
|---|---|---|---|
| Control | 6 | 4/6 66% | 0-7 |
| 0,2 mg/kg | 7 | 5/7 71% | 0-7 |
| 0,8 mg/kg | 5 | 2/5 42% | 0-2* |
| 3,2 mg/kg | 5 | 2/5 42% | 0-3* |

### Oral administration route

The effect of scorpion venom on an mammary adenocarcinoma model implanted in Balb c mice was evaluated. Four doses (6 mg/kg, 12.5 mg/kg, 25 mg/kg and 50 mg/kg) for the treatment groups were used. Controls were given a saline solution and all administrations were through oral route for 35 days. Tumor growth was monitored for 35 days.

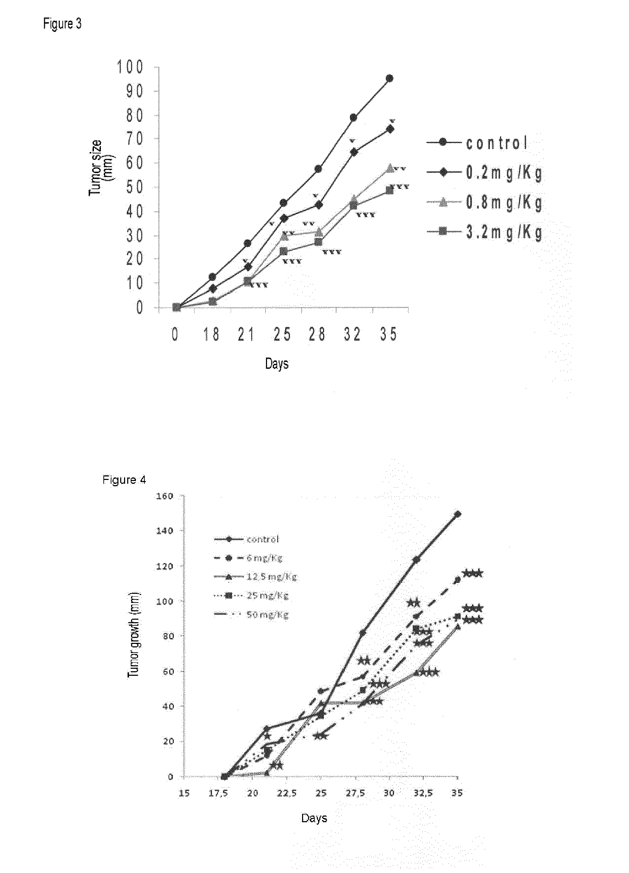

The experimental groups administered with scorpion venom showed significant tumor growth inhibition (p < 0.05) compared to the control group (Figure 4.) The experimental groups showed a dose-response ratio during the 35 days of evaluation as to tumor progression retardation.

**Table XX. Mean survival time of experimental groups implanted with 10⁶ S-180 mice cells intraperitoneally and treated with R. junceus scorpion venom given orally.**

| Dose | Control | 6 mg/kg | 12,5 mg/kg | 25 mg/kg | 50 mg/kg |
|---|---|---|---|---|---|
| Survival time (days) | 20 | 20,5 | 24 | 22,5 | 20 |

**Table XXI. Survival time among experimental groups implanted with 10⁶ S-180 mice cells intraperitoneally and treated with R. junceus scorpion venom given orally.**

| Dose | Control | 6 mg/kg | 12,5 mg/kg | 25 mg/kg | 50 mg/kg |
|---|---|---|---|---|---|
| Survival, % | 0 | 26 | 37 | 22 | 0 |

The 12.5-mg/kg dose gave a longer mean survival time (24 days), followed by the 25-mg/kg dose (22.5 days) and the 6-mg/kg dose (20.5 days), while the mean survival time of controls was 20 days (Table XXI.) Similarly, the 12.5-mg/kg dose evidenced the highest survival percents by the time at which 100% of mice in the control group had died (Table XXI), while the 6-mg/kg and 25-mg/kg doses showed 26% and 22%, respectively. Notwithstanding these results, the longer survival time and the survival percents among the three experimental groups (6 mg/kg, 12.5 mg/kg and 25 mg/kg) were not statistically significant. The 50-mg/kg dose showed similar values to those of controls in all cases.

### Assay on the antiproliferative activity of fractions obtained from molecular exclusion chromatography

The effect of protein fractions on cell growth was determined in a similar way as that in Example 6. The study used 2 tumor cell lines: HeLa (human cervix carcinoma) and A549 (human lung carcinoma) and the normal MRC-5 cell line (human lung fibroblasts.) Fractions were at a final 9 µg/mL-600 µg/mL concentration in wells.

The cytotoxity evaluation of cell line fractions evidenced inhibited growth of tumor cells and little toxicity in normal cells for the LB-03 and LB-04 fractions. Table XXII shows the CC₅₀ for each of the purification fractions.

**Table XXII. Relative molecular weights and mean cytotoxic concentration of fractions from molecular exclusion chromatography in tumor and normal cell lines.**

| *R. junceus* venoms fractions | Relative molecular weights^{*} | Mean cytotoxic concentration (CC₅₀) (µg/mL) | | |
|---|---|---|---|---|
| | | Hela | A549 | MRC-5 |
| LB-01 | 30 kDa-72 kDa | 835,3 | 420 | 245,1 |
| LB-02 | 14 kDa- 30 kDa | 208,7 | 250 | 276,9 |
| LB-03 | 4 kDa - 8 kDa | 140,1 | 116,6 | 439,2 |
| LB-04 | ≤4 kDa | 423,6 | 283 | 613,9 |

| | | | | |
|---|---|---|---|---|
| *Relative molecular weights determined from the log PM vs VₒVₑ. calibration curve. MW: Molecular weights of pattern proteins. Vₒ: Dead volume of column. Vₑ: Elusion volume for each protein peak. | | | | |

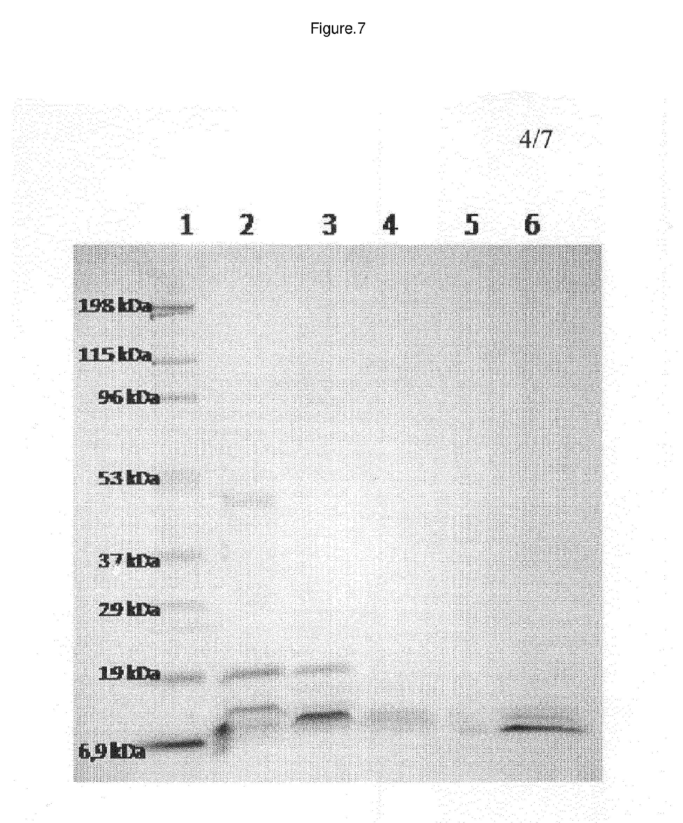

Fractions obtained from FPLC molecular exclusion chromatography were run in polyacrilamide gel electrophoresis with a 4-20% gradient to verify their protein composition, which appears in Fig. 7.

The LB-03 and LB-04 fractions have a low molecular weight protein composition and, additionally, they were the fractions which showed higher cytotoxicity on tumor cells (Hela and A549) and low toxicity on normal cells (MRC-5.)

These fractions were re-purified through high-resolution liquid chromatography using a C18 reverse-phase analytical curve. Two solutions were used as mobile phase: Solution A (0.12% trifluoroacetic acid (TFA) in water) and Solution B (0.10% TFA in acetonitrile.) Fraction component elusion was done with gradual gradients from 0 to 70% of Solution B for 70 minutes and at a 0.5 ml/min flow and the fraction detection wavelength was 220 nm. The obtained pure peptides in tumor (Hela) and normal (CHO) cells were evaluated in a way similar to that described in Example 6 and their molecular weight and protein sequence were determined through mass spectrometry built in a high-resolution liquid chromatography device.

Eight peptides (RjLB-01, RjLB-03, RjLB-04, RjLB-05, RjLB-07, RjLB-08, RjLB-09 and RjLB-14) with high cytotoxic activity on tumor cells and low toxicity on normal cells were obtained. These results corroborate what was observed with whole venom and confirm the preferential toxicity of venom components on tumor cells.

Based on the purification of active components, their proportional presence in venom was determined (Table XXIII.)

**Table XXIII. Proportion of active principles in venom composition and range of in vitro biological activity on tumor cells.**

| **Active principle** | **Proportion of venom, %** | ***In vitro* biological activity range (%)** |
|---|---|---|
| RjLB-01 | 1,5-20 | 0.945-1,89 |
| RjLB-03 | 8-9 | 4,5-9 |
| RjLB-04 | 0,5-1,0 | 0,445-0,89 |
| RjLB-05 | 0,5-0,7 | 0,33-0,71 |
| RjLB-07 | 0,5-0,8 | 0,5-1,0 |
| RjLB-08 | 0,5-1,0 | 1,5-3,0 |
| RjLB-09 | 0,3-0,6 | 3,65-4,1 |
| RjLB-14 | 0,4-0,8 | 3,5-6,0 |

| | | |
|---|---|---|
| (pag. 24) | | |

The molecular weights obtained for the RjLB-01, RjLB-03 and RjLB-04 peptides were 908 Da, 1964 Da and 4748,14 Da, respectively. The obtained sequences were compared to scorpion venom peptide data bases and no homology with previously described peptides was found, which is additional evidence of the novelty of results.

Another result from this invention is the formulation obtained as follows: Fifty to one hundred *Rhopalurus junceus* species scorpions were taken and their venom extracted by way of electric stimulation and diluted in 10-20mL distilled water. It was then clarified through 10 000 rpm centrifugation for 15 minutes to get rid of components such as mucus and cell debris. At the same time, their proteins were determined using the Lowry method and the result was a concentration of 5-15 mg/mL. Then the scorpion venom was diluted conveniently with distilled water as the only excipient to obtain a formulation whose concentration range was 0.05-0.1 mg/mL.

### Brief description of figures

Figure 1 shows the behavior of codeine (positive control), a central analgesic drug whose mean life fluctuates within 2-3 hours, which coincides with our results.
Figure 2 shows 1.5% agarose gel electrophoresis of DNA extracted from the A549 and Hela tumor cell lines. Venom was given at an end concentration of 0.5 mg/mL per well. DNA from each cell line was extracted after 24 and 48 hours.
   Track M: Lambda DNA/Hind III molecular weight marker.
   Track 1-3: A549 cell line; 1: DNA control without venom; 2: DNA extracted after 24 hours; 3: DNA extracted after 48 hours.
   Track 4-6: Hela cell line; 4: DNA control without venom; 5: DNA extracted after 24 hours; 6: DNA extracted after 48 hours.
Figure 3 shows the kinetics of tumor growth in the experimental groups. Tumor growth was monitored for 35 days. Tumor growth inhibition depended on the venom doses that were given. The statistical significance as per control was *p < 0.05; **p < 0.01, ***p < 0.001.
Figure 4 shows the kinetics of tumor growth in the experimental groups. Tumor growth was monitored for 35 days. Tumor growth inhibition depended on the venom doses that were given. The statistical significance as per control was *p < 0.05; _{**}p < 0.01, ***p < 0.001.

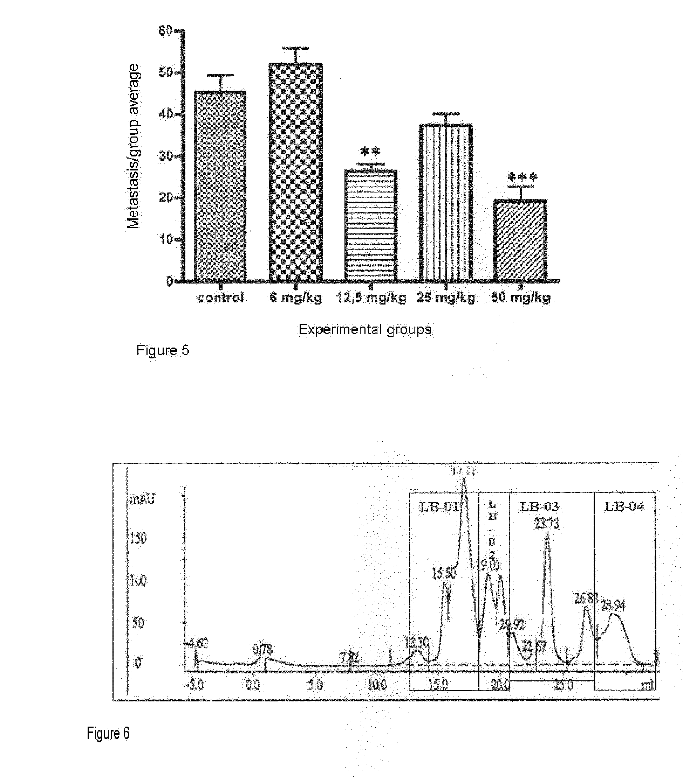

Figure 5 shows the effect t of oral administration of scorpion venom on the incidence of experimental lung metastases. Statistically significant difference: **p < 0.01, ***p < 0.001.
Figure 6 shows the *R. junceus* scorpion venom chromatographic profile in low-pressure liquid chromatography using a 12 HR10/30 Superose molecular exclusion column.
Figure 7 shows the polyacrylamide gel electrophoresis in reductive conditions. Fractions obtained from purification were applied using low-pressure liquid chromatography. Track 1: Molecular Weight Marker; Track 2: Whole Venom; Track 3: LB-01 Fraction; Track 4: LB-02 Fraction; Track 5: LB-03 Fraction; Track 6: LB-04 Fraction.

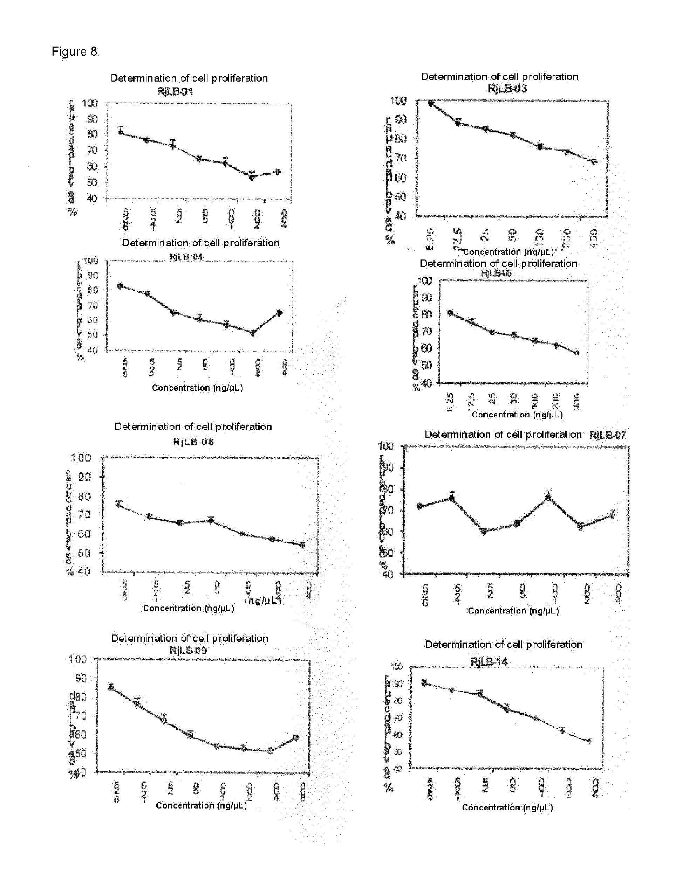

Figure 8 shows the concentration-response curve for pure peptides which were obtained from the re-purification of evaluated fractions. The evaluation was performed on Hela (human cervix carcinoma) tumor cells. The concentrations used were 6.25 µg/mL-400 µg/mL.

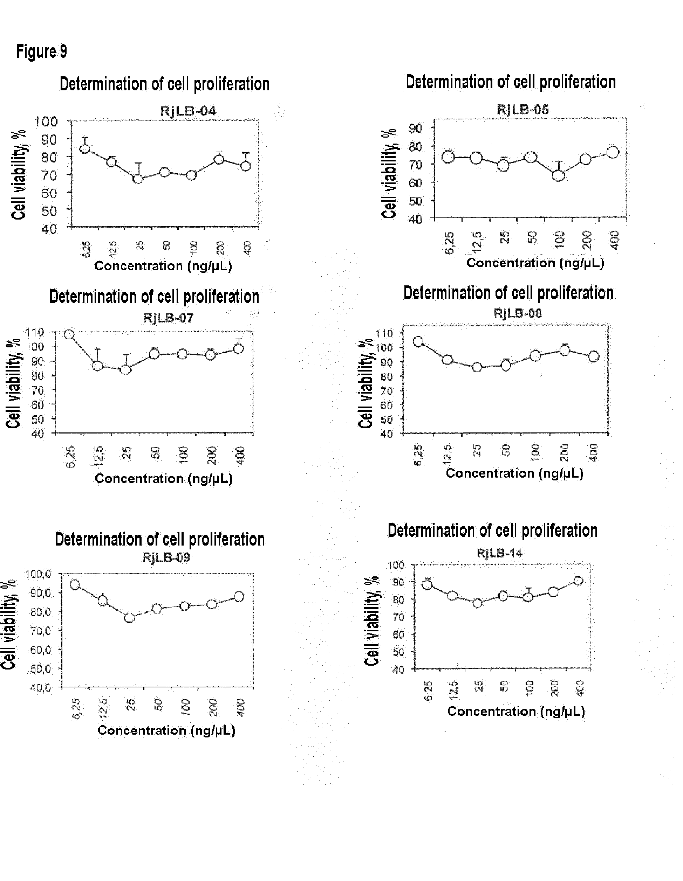

Figure 9 shows the concentration-response curve for pure peptides which were obtained from the re-purification of evaluated fractions. The evaluation was performed on CHO (Chinese Hamster Ovarium) non-tumor cells. The concentrations used were 6.25 µg/mL-800 µg/mL.

### Example 1 Acute inflammation model of croton oil-induced auricular edema in mice.

OF-1 male mice weighing an average 18-22 g from the National Center for Laboratory Animal Production (CENPALAB) were used. The method described by CYTED (Ibero-American Programme for Science and Technology Development, Lima, November of 1996) was used. A 0.5%-acetone croton oil solution was used. Dexamethasone was dissolved in 0.5% CMC.

### Example 2 Cotton ball implantation model in rats

Sprague-Dawley rats of 180g body weight from the National Center for Laboratory Animal Production (CENPALAB) were used.
The method for cotton ball implantation from CYTED (Ibero-American Programme for Science and Technology Development, Lima, November of 1996) was carried out. Inflammation is the tissue response to damage. This includes enzyme involvement and activation, mediator release, fluid extravasation, cell migration and tissue rupture and repair (Vane, 1995.) It is well known that the anti-inflammatory effect may result from a series of chemical agents and that there is a slight correlation between their pharmacological activity and chemical structure (Sertie, 1990.) This, combined with the complexity of the inflammatory process, requires the use of various experimental models for carrying out a pharmacological assay. The cotton pellet granulose model in rats is a frequently used assay to study the effect on chronic inflammation conditions (Elieter, 1999.) Three inflammation phases after pellet implantation have been demonstrated. The last phase is that of cell proliferation occurring between the third and sixth days. This phase may be inhibited by anti-inflammatory steroids such as dexamethasone and by non-steroidal anti-inflammatory drugs (Sw, 1972.)

The animals were sacrificed 7 days after the implantation of cotton plugs through the established euthanasia method and the pellets were extracted and studied. The pellet's end- and initial-weight difference was considered to be the granulomatose tissue produced.

### Example 3 Inflammatory angiogenesis model

Sprague-Dawley male rats from CENPALAB with 180-240 g body weight were used. Inflammatory angiogenesis is a complex process involving a series of different but basically similar molecular mechanisms which develop in an angiogenic cascade. Six days after granuloma formation was induced, the granuloma was well defined and palpable in the control group, which coincides with the results reported by Primelles in 2001. In the case of animals given venom doses, the granulomas were smaller at touch and a macroscopic observation of them showed less-defined ends and they were not so attached to the epidermis.

### Example 4. Analgesic activity

### ● Model of acetic acid-induced contorsions in mice

The method described in CYTED was followed.

OF1 male mice weighing an average 20-25g from the National Center for Laboratory Animal Production (CENPALAB) were used.

### Intraperitoneal route administration

Doses of 1-, 2.5- and 7.5-mg/kg *R. junceus* venom were inoculated intraperitoneally. Thirty minutes later, the experimental animals were administered 0.1 mL of a 3-percent acetic acid solution intraperitoneally and the number of contorsions/animal was recorded for 10 minutes beginning with the first contorsion observed.

### Oral route administration

Doses of 1-, 5-, 10-, 15-, 20- and 50-mg/kg *R. junceus* venom were administered orally. One hour later, the experimental animals were administered 0.1 mL of a 3-percent acetic acid solution intraperitoneally and the number of contorsions/animal was recorded for 10 minutes beginning with the first contorsion observed.

### Example 5 Evaluation of antioxidative activity.

The *in vitro* anti-oxidative activity by *Rhopalurus junceus* venom was evaluated through the DNA protective effect in Copper-Phenanthroline and Bleomycin-Iron systems oxidative processes. The venom showed anti-oxidative activity as it protected DNA from peroxidation processes.

The Copper-Phenanthroline and Biomycin-Iron systems showed that the *Rhopalurus junceus* derivative in the above-described formulations has no pro-oxidative effect on DNA. The absorbance values obtained with various *Rhopalurus junceus* derivative concentrations are lower than those obtained with the positive control. Tables XVI and XVII show the results from such evaluation.

### Example 6. In vitro cytotoxicity in a panel of tumor and normal cells

The effect of venom on cell growth was seen in the MTT assay, which measures cell proliferation resulting from the metabolic reduction of this (yellow) tetrazolium salt by mitochondrial dehydrogenated enzymes. The resulting compound (blue formazan) can be made soluble and quantified spectrophotometrically

Nine tumor cell lines were used in the study: HeLa (human cervix carcinoma), HEp-2 (human laryngeal epidermoid carcinoma), NCI-H292 (human lung mucoepidermoid carcinoma), A549 (human lung carcinoma), U937 (human histyocitic lymphoma), L929 (murine fibrosarcoma), S-180 (murine sarcoma), and F311 (murine breast sarcoma.) Equally, 3 normal cell lines were used: MRC-5 (human lung fibroblasts), Vero (African green monkey normal kidney cells), and MDCK (normal dog kidney cells.) Peritoneal macrophages (extracted from Balb/c mice peritonea) and lymphocytes (extracted from Balc/c mice spleens) were also evaluated. Cells were grown within culture flasks in a minimal essential medium (MEM) or in a RPMI-1640 medium, depending on cell culture characteristics, and added 2 mM glutamine, non-essential amino acids, 10% bovine fetal serum (BFS), and 100 IU-100 µg/mL penicillin-streptomycin. Each one was incubated in a humid atmosphere at 37°C and 5-percent CO₂ till a single-layer was formed. Each cell line was separated by way of a 0.25% trypsin-EDTA solution and prepared at a 2 x 10⁵ cell/mL concentration after being counted in a Neubauer chamber.

The assay was done in 96-well, flat-bottom cell culture polystyrene dishes *(Coming Inc. costar^{R}.*) 50 µL of each cell line was poured into each well and incubated at a 5-percent CO₂ and 37°C atmosphere for 24 hours. After such time, 50 µL medium containing previously dissolved venom was added. Final venom concentrations were 0.1 mg/mL, 0.25 Mg/mL, 0.5 mg/mL, 0.75 mg/mL and 1 mg/mL in wells. All final cell line concentrations were 10⁴ cells/well. The bovine fetal serum (BFS) was used in the medium at 10%.

Dishes were incubated again at a 5-percent CO₂ and 37°C atmosphere for three days. After such time, a 10 µL MTT sterile solution (5 mg/mL tetrazolium salts in sterile PBS) was added to each well and incubated under the same conditions for 4 hours. Finally, the medium was poured off and a 200 µL/well dimethyl sulfoxide (DMSO) solution was added and incubated at 37°C for 30 minutes in a humid atmosphere. The optical density (OD) was read on a Revelation Dynex Technologies ELISA MRX microdish reader at 560 nm with 630 nm as reference. Each fractional concentration was done three times and the assay was performed four times.

For the concentration-response curves analysis, the cell proliferation percent was graphically obtained from the formula (1-OD (sample)/OD (control)) X100 against the different venom concentrations. Mean cytotoxic concentration was expressed as CC₅₀, which is the venom concentration that provokes a 50-percent decrease in the number of viable cells (MTT absorbance) compared to untreated controls and appears in Table XV for each of the evaluated cells.

### Example 7. Apoptosis study

The study for determining apoptosis was done through DNA fragmentation. The Hela and A549 cell lines were used. Results are shown in Figure 2. The characteristic DNA fragmentation of apoptosis was observed in the corresponding tracks of Hela cells treated with venom, while a single intact chromosomal DNA band in control wells and in the A549 cell line was observed. These results show that scorpion venom may induce cell death through both mechanisms -apoptosis and necrosis.

### Example 8. Antitumor activity in solid tumors F311 breast adenocarcinoma experimental model . Intraperitoneal administration route

The effect of scorpion venom on a mammary adenocarcinoma model implanted in Balc c. mice was evaluated. Three doses (0.2 mg/kg, 0.8 mg/kg and 3.2 mg/kg) were used for the treatment groups. Controls were administered a saline solution intraperitoneally. Tumor growth was monitored for 35 days. Fifty days after the implantation of the tumor the animals were sacrificed, their lungs were extracted and pulmonary metastasis was searched for.

The experimental groups treated with scorpion venom showed significant tumor growth inhibition (p < 0.05) compared to the control group (Figure 3.) The experimental groups showed a dose-response ratio during the 35 days of evaluation as to tumor progression retardation. The significant reduction of tumor growth in the treated groups indicates that *R. junceus* scorpion venom has an antitumor effect as it influences tumor growth, at least during the evaluation period.

### Example 9 F311 breast adenocarcinoma. Experimental model. Oral administration route

The effect of scorpion venom in a breast adenocarcinoma model that was injected in Balb c mice was evaluated. Four doses (6mg/kg, 12,5 mg/kg, 25 mg/kg and 50 mg/kg) were used for the treatment groups, the control group was administered a saline solution orally. Tumor growth was monitored for 35 days.
The experimental groups treated with scorpion venom (12, 5 mg/kg, 25 mg/kg and 50 mg/kg) showed significant tumor growth inhibition (p<0.05) when compared to the control group (Figure 4.) The significant decrease in tumor progression that was found in the treated groups demonstrates the antitumor effect of the *R. junceus* scorpion venom, as it affected tumor growth at least during the evaluation period.

### Example 10. Antihumor activity in ascitic tumors. Experimental sarcoma model (S-180)

The effect of scorpion venom in a murine sarcoma model implanted in the peritoneum of NMRI mice was evaluated. Four doses (6 mg/kg, 12.5 mg/kg, 25 mg/kg and 50 mg/kg) for treatment groups were used. The control group was given a saline solution. The venom was administered daily 24 hours after the implantation of the tumor until all experimental groups died. Table XX shows the survival time results with the treated animals and controls. Table XXI shows the survival percents among experimental groups.

### Example 11. Purification and identification of venom proteins as active principles.

In order to separate whole protein content, the whole venom was dissolved in 0,1 M ammonium acetate (NH₄Ac) and centrifuged at 10 000 rpm for 15 minutes. The supernatant was put in an AKTA FPLC (Amersham Pharmacia Biotech) low-pressure liquid chromatography equipment with a 12 HR 10/30 Superose filtration gel column measuring 10 x 300 mm. The column was balanced with 0.1M NH₄Ac and the material mixed with same solvent at a flow rate of 0.5 mL/min. Absorbance was monitored at an optical density (OD) of 280 nm for 72 minutes. The 75 HR 10/20 Superose molecular exclusion column was calibrated with a pattern protein kit including ribonuclease A (13.7 Kda), chemotripsinogen (25 Kda), ovoalbumin (43 Kda), albumin (67 Kda) and blue dextrane 2000. A pattern curve was drawn to determine the relative molecular weights of the various protein fractions obtained during chromatography. The results from chromatographic runs appear in Figure 6.

### Example 12 Clinical studies about the formulation given to human cancer patients

The purpose of the study was to assess life quality in cancer patients. The subjects included in the study were patients of both sexes with histologically confirmed cancer at any stage. A document was written which stated the informed consent of patients and a summary of the clinical record issued by the treating oncologist. The document included an evaluation of the clinical condition of patients as followed by their physicians in the hospital during their clinical evolution. Follow-up periodicity was no less than two months. Follow-up took place for a year. The formulation was prepared in 40-mL flasks with 0.05-0.1 mg/mL concentrations. Flask content was diluted in distilled water used as excipient to complete 1 litter. The product was administered daily by oral route and the recommended doses depended on patient condition and histological diagnosis.
A total of 100 patients were included in the study and the main evaluated cancer locations were breast, prostate, colon, lung, brain and pancreas. The administration of the product did not cause any adverse reaction at any moment during treatment, which coincided with observations during pre-clinical studies. All cases improved their quality of life as evidenced by an improvement in their main clinical variables such as less or no dysnea and coughing in all lung cancer patients. Many patients showed better radiological test results in the form of lesion stabilization in some cases and disappearance in others. Additionally, hematological variables were stabilized and pain and swelling reduced. Over 50% of the patients treated with the invented formulation for a year had a longer survival time than the one estimated for some of the studied conditions.

### Example 13.

### Clinical studies of the formulation in human cancer patients..

The objective of the study was the evaluation of life quality in cancer patients. Patients of both sexes with histological confirmation of cancer in any stage were used to be included as study individuals. A document was prepared which contained the patient's consent act and the summary of the clinical record issued by the primary health care oncologist.
The evaluation of the clinical behavior of patients was included in the document and the evaluation was followed up through the clinical progress and conducted by the specialists from the patients' origin hospitals. The periodicity of the follow-up was of at least two months. The follow-up was carried out during 1 year. The formulation was prepared in bottles with a volume of 40 mL with concentrations between 0.05-0.1 mg/mL. The flask was diluted in distilled water used as excipient until completing 1 liter. The product was administered on a daily basis through the oral route and the recommended dose depended on the patient's stage and histological diagnosis.

A total of 100 patients were included in the study and the main locations for cancer were, breast, prostate, colon, lung, brain and pancreas. In no case the administration of the product caused adverse reactions during the treatment which coincided with what was observed in the preclinical studies. In all cases it was observed an increase in life quality including an improvement of the main clinical variables such as decrease or disappearance of dyspnea and coughing in all cases of lung cancer patients. In many patients it was verified an improvement in the x-ray tests results as their lesions became stable in some case and in others lesions disappeared. In addition to this, the stabilization of the hematological variables was achieved and there was a reduction of pain and inflammation. In over 50 % of the patients treated for 1 year with the formulation object of the invention, the survival period estimated for some of the pathologies studied was exceeded.

### Example 14. Vidatox ® 30 CH clinical studies

**VIDATOX ® 30 CH** is a homeopathic biotherapeutical drug developed at the Homeopathy Laboratory of LABIOFAM. Its active ingredient is *Rhopalurus junceus* scorpion venom in a 30 centesimal dilution. It comes in the form of drops in a 33% alcohol vehicle.
The studies carried out in 174 patients, of both sexes, having specific cancer treatments demonstrated the adjuvanting effect of **VIDATOX ® 30 CH** for treating cancer in different body location, (lung, prostate, breast, colon, pancreas, t. lymphoid, brain, rectum etc.) Its results show that 96 % of patients administered with the product had a survival of over 12 months, that 90% of the patients reported improvement in their clinical symptoms seen by physicians and that among these 63 patients, pain (as the predominant symptom) for 62 % of them evolved to a moderate form, which did not require relief treatment, that the 27 % of them reported no pain and none of them reported adverse reactions to the treatment.

## Claims

1. Peptide called RjLB-01, obtained from scorpion venom and **characterized by** having 544.42 Da molecular weight and SEQ. DI No. 1 amino acid sequence.

2. Peptide called RjLB-03, obtained from scorpion venom and **characterized by** having 1964.0 Da molecular weight and SEQ. DI No. 2 amino acid sequence.

3. Peptide called RjLB-04, obtained from scorpion venom and **characterized by** having 4748.14 Da molecular weight SEQ. DI No. 3 amino acid sequence.

4. Peptide called RjLB-05, obtained from scorpion venom and characterized having 908.0 molecular weight of Da and SEQ. DI No. 4 amino acid sequence.

5. Peptide called RjLB-07, obtained from scorpion venom and **characterized by** having 707.03 Da molecular weight and SEQ. DI No. 5 amino acid sequence.

6. Peptide called RjLB-08, obtained from scorpion venom and **characterized by** having 712.42 Da molecular weight and SEQ. DI No. 6 amino acid sequence.

7. Peptide named RjLB-09, obtained from scorpion venom and **characterized by** having 1203.44 Da molecular weight and SEQ. ID No. 7 amino acid sequence.

8. Peptide called RjLB-014, obtained from scorpion venom and **characterized by** having 5930. 45 Da molecular weight and SEQ. DI No. 8 amino acid sequence.

9. Pharmaceutical compositions that are obtained from *Rhopalurus junceus scorpion venom,* **characterized by** having peptides as active principles with high cytotoxic activity on tumor cells, mixed with distilled water as excipient

10. Pharmaceutical compositions as per claim 9 which are processed-homeopathically.

11. Pharmaceutical compositions as per claim 9, containing at least one peptide according to claims 1-8, which has antitumor activity, and such selected peptide having any of the described amino acid sequence.

12. Pharmaceutical compositions as per claim 11 **characterized by** having a mix of peptides contained in *Rhopalurus junceus* scorpion venom with the following concentrations: RJL-01, at a 1,5-2,0% range; RJLB-03, at a 8-9% range; RJLB-07, in a 0,5-0,8% range; RJLB-08 at a 0,5-1,0% range; RJLB-09, at a 0,3-0,60% range; RJLB-14, at a 0, 4-0-8% range, diluted in 10-20 mL distilled water.

13. Pharmaceutical compositions, as per claim 12, processed homeopathically.

14. Pharmaceutical compositions, as per claims 9, 10,11, 12 and 13, used in formulations to be administered by oral, topical, parenteral, rectal, vaginal and aerosol routes.


SEQUENCE LISTING

<110> Grupo Empresarial LABIOFAM

<120> Peptides from the venom of the Rhopalurus junceus scorpion and
pharmaceutical compostion.

<130> 01-00001

<140> PCT/CU2011/000006
<141> 2011-09-27

<150> CU2010-0186
<151> 2010-09-27

<160> 8

<170> PatentIn Ver. 2.1

<210> 1
<211> 10
<212> PRT
<213> Rhopalurus junceus

<400> 1
Arg Thr Gly Ser Leu Leu Thr Leu Ala Arg
  1               5                  10

<210> 2
<211> 17
<212> PRT
<213> Rhopalurus junceus

<400> 2
Asp Leu Phe Asp Glu Leu Asp Pro Gln Lys Asp Glu Leu Asp Gln Gly
  1               5                  10                  15

Ser

<210> 3
<211> 43
<212> PRT
<213> Rhopalurus junceus

<400> 3
Phe Glu Leu Lys Asp Leu Glu Gly Gln Thr Leu Tyr Leu Lys Asp Leu
  1               5                  10                  15

Glu Gly Lys Thr Cys Ser Gly Gly Glu Leu Asp Lys Glu Glu His Cys
             20                  25                  30

Gly Gly Asn Glu Leu Asp Ser Gln Gly Phe Asn
         35                  40

<210> 4
<211> 8
<212> PRT
<213> Rhopalurus junceus

<400> 4

Met Leu Leu Asp Asn Val Gly Phe
1               5

<210> 5
<211> 7
<212> PRT
<213> Rhopalurus junceus

<400> 5
Asp Met Gly Ala Leu Leu Ser
1               5

<210> 6
<211> 17
<212> PRT
<213> Rhopalurus junceus

<400> 6
Phe Asp Glu Thr Ala Lys Asp Glu Leu Leu Gly Phe Leu Gly Leu Phe
1               5                   10                  15

Asn

<210> 7
<211> 12
<212> PRT
<213> Rhopalurus junceus

<400> 7
Leu Pro Cys Ala Leu Asn Asp Gly Gly Leu Cys Lys
1               5                   10

<210> 8
<211> 50
<212> PRT
<213> Rhopalurus junceus

<400> 8
Val Met Gln Asp Gln Tyr Leu Ile Ile Asn Gly Val Trp Ser Ile Val
1               5                   10                  15

Phe Phe Leu Glu Val Ser Ile Leu Val Ile His Ile Phe Leu Leu Arg
            20                  25                  30

Glu His Glu Val Phe Val Val Arg Arg Gly Asp Glu Thr Ala Lys Asp
        35                  40                  45

Glu Leu
    50